(19) Europäisches Patentamt
European Patent Office
Office européen des brevets

(11) Veröffentlichungsnummer : **0 486 897 B1**

(12) # EUROPÄISCHE PATENTSCHRIFT

(45) Veröffentlichungstag der Patentschrift : **03.08.94 Patentblatt 94/31**

(51) Int. Cl.$^5$ : **C07C 235/20,** C07C 235/84, C08F 20/60, C09J 133/24

(21) Anmeldenummer : **91119041.1**

(22) Anmeldetag : **08.11.91**

(54) **Ungesättigte Phenonderivate und ihre Verwendung als Haftklebstoffe.**

(30) Priorität : **22.11.90 DE 4037079**

(43) Veröffentlichungstag der Anmeldung : **27.05.92 Patentblatt 92/22**

(45) Bekanntmachung des Hinweises auf die Patenterteilung : **03.08.94 Patentblatt 94/31**

(84) Benannte Vertragsstaaten : **BE CH DE ES FR GB IT LI NL SE**

(56) Entgegenhaltungen :
**DE-A- 3 844 445**
**FR-A- 2 203 807**
**US-B- 408 487**

(73) Patentinhaber : **BASF Aktiengesellschaft**
**Carl-Bosch-Strasse 38**
**D-67063 Ludwigshafen (DE)**

(72) Erfinder : **Barwich, Juergen, Dr.**
**Triftbrunnenweg 27**
**W-6730 Neustadt (DE)**
Erfinder : **Rehmer, Gerd, Dr.**
**Koenigsberger Strasse 1**
**W-6711 Beindersheim (DE)**
Erfinder : **Bott, Kaspar, Dr.**
**Werderstrasse 57**
**W-6800 Mannheim 1 (DE)**

EP 0 486 897 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ungesättigte Phenonderivate der allgemeinen Formel I

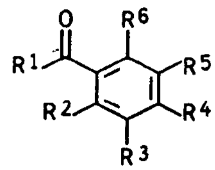

I

in der die Variablen folgende Bedeutung haben:

$R^1$ eine $C_1$- bis $C_4$-Alkylgruppe, eine Cyclopropyl-, -pentyl- oder -hexylgruppe, eine Indanonyl- oder Tetralonylgruppe, die Phenylgruppe, eine Phenylgruppe, deren Wasserstoffatome teilweise oder vollständig durch eine $C_1$- bis $C_4$-Alkyl-, -Alkoxy- oder -Thioalkylgruppe ersetzt sind, oder gemeinsam mit $R^2$ oder $R^6$ eine Ethylen- oder Propylenbrücke;

$R^2$ bis $R^6$ ein Wasserstoffatom, eine $C_1$- bis $C_4$-Alkyl, -Alkoxy- oder -Thioalkylgruppe, wobei $R^3$, $R^4$ und $R^5$ zusätzlich für eine Hydroxylgruppe stehen können, $R^2$ oder $R^6$ zusätzlich gemeinsam mit $R^1$ eine Ethylen- oder Propylenbrücke bilden können und mindestens einer, höchstens jedoch drei der Reste $R^2$ bis $R^6$ für eine Gruppe der allgemeinen Formel II

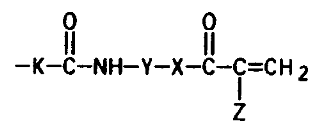

II

oder III

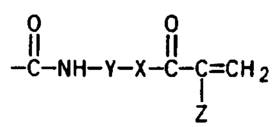

III

stehen, in denen die Variablen folgende Bedeutung haben:

K eine $C_1$- bis $C_{10}$-Alkylen-, eine 1 bis 2 Sauerstoff- oder Schwefelatome enthaltende $C_1$- bis $C_{10}$-Alkylengruppe

Y eine geradlinige oder verzweigte $C_1$- bis $C_{10}$-Alkylengruppe oder eine durch Carboxyl-, Carboxylatanion-, $C_1$- bis $C_4$-Carbonsäurealkylester- oder Hydroxygruppen substituierte $C_1$- bis $C_{10}$-Alkylengruppe

X -NH- oder -(N-Alkyl)- mit 1 bis 4 C-Atomen

Z Wasserstoff oder eine $C_1$- bis $C_4$-Alkylgruppe

Weiterhin betrifft die vorliegende Erfindung ein Verfahren zur Herstellung der Monomeren, sowie Polymerisate, welche die ungesättigten Phenonderivate enthalten und die Verwendung der Polymerisate als Haftklebestoffe.

Ungesättigte Phenonderivate des allgemeinen Aufbaus

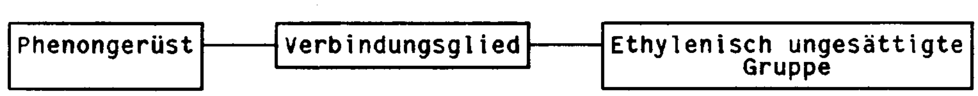


sind bereits bekannt. Polymerisate dieser Monomeren eignen sich besonders zur Verwendung für Haftklebestoffe, da sie durch Bestrahlung mit UV-Licht vernetzbar sind, d.h. nach Bestrahlung eine höhere innere Festigkeit aufweisen. Die Haftung auf Substraten wird durch polare Gruppen bewirkt.

In der US 3 214 492 werden Verbindungen der allgemeinen Formel (VI) beschrieben

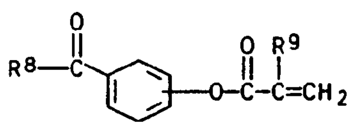

VI

in der

$R^8$ -$CH_3$ oder -$C_6H_5$ und

$R^9$ -H oder -$CH_3$

bedeuten.

Ähnliche Acryloxy- oder Methacryloxygruppen enthaltende Acetophenon- oder Benzophenonderivate sind aus der US-A-3 429 852 bekannt.

Die DE-A-28 18 763 betrifft Verbindungen der allgemeinen Formel VII

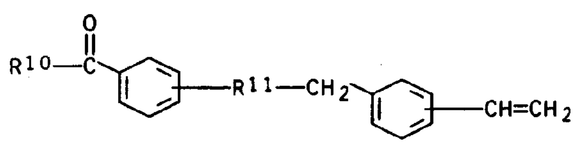

VII

in der

$R^{10}$ -$C_nH_{2n+1}$ mit n = 1 bis 3 oder -$C_6H_5$,

$R^{11}$ -O-,

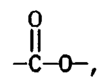, 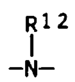 oder 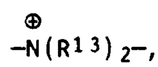,

$R^{12}$ -H oder -$C_nH_{2n+1}$ mit n = 1 bis 8 und

$R^{13}$ -$C_nH_{2n+1}$ mit n = 1 bis 4 bedeuten.

In der DE-A-38 20 464 und DE-A-38 20 463 werden Monomere beschrieben, die durch Umsetzung eines Phenonderivats, welches am Phenylkern durch eine Hydroxylgruppe substituiert ist, mit einem ungesättigten Isocyanat erhalten werden.

Aus der DE-A-38 44 445 und DE-A-38 44 444 sind Monomere bekannt, die durch Umsetzung eines Phenonderivats, welches ebenfalls am Phenylkern durch eine Hydroxylgruppe substituiert ist, mit einem ungesättigten aktivierten Carbonsäureester hergestellt werden.

Aus der älteren deutschen Anmeldung P 40 07 318.1 sind Phenonderivate bekannt, die durch eine Amidomethylierung erhalten werden.

Die ungesättigten Phenonderivate sollen eine möglichst hohe photochemische Reaktivität, thermische Stabilität und Hydrolysebeständigkeit aufweisen. Desweiteren sollen die Polymerisate, welche die ungesättigten Phenonderivate enthalten, gut auf Substraten haften. Von besonderer Bedeutung für die Verfügbarkeit der ungesättigten Phenonderivate ist, daß sie durch ein möglichst einfaches Herstellungsverfahren erhältlich sind. Für die Verwendung der ungesättigten Phenonderivate in Copolymerisaten ist ein gutes Copolymerisationsverhalten Voraussetzung.

Aufgabe der vorliegenden Erfindung waren ungesättigte Phenonderivate, die möglichst alle oben genannten Anforderungen erfüllen.

Die Aufgabe wurde gelöst durch ungesättigte Phenonderivate gemäß Anspruch 1.

Besonders geeignete ungesättigte Phenonderivate der Formel I sind solche, bei denen $R^1$ für eine Phenylgruppe oder eine $C_1$- bis $C_4$-Alkylgruppe steht. Bei den Resten $R^2$, $R^3$, $R^4$, $R^5$ und $R^6$ handelt es sich bevorzugt um ein Wasserstoffatom oder eine $C_1$- bis $C_4$-Alkyl- oder -Alkoxygruppe, bei mindestens einer, höchstens jedoch drei der Reste $R^2$-$R^6$ für eine Gruppe der allgemeinen Formel II oder III steht. Das ungesättigte Phenon enthält bevorzugt nur eine Gruppe der allgemeinen Formel II oder III, insbesondere enthält es diese Gruppe als Substituent $R^4$. Besonders bevorzugt haben $R^2$, $R^3$, $R^5$ und $R^6$ die Bedeutung von Wasserstoffatomen.

In der allgemeinen Formel II oder III steht K bevorzugt für eine $C_1$- bis $C_6$-Alkylengruppe, oder eine 1 oder 2 Sauerstoffatome enthaltende $C_1$- bis $C_6$-Alkylengruppe. Besonders bevorzugt steht K für eine $C_1$- bis $C_4$-Alkylengruppe oder $C_1$- bis $C_4$-Oxyalkylengruppe, bei der das Sauerstoffatom direkt an den Phenylring gebunden ist.

Y steht bevorzugt für eine (geradlinige oder verzweigte) $C_1$- bis $C_6$-Alkylengruppe oder eine durch Carboxyl-, bzw. Carboxylatanion-, $C_1$- bis $C_4$-Carbonsäurealkylester- oder Hydroxygruppen substituierte $C_1$- bis $C_6$-Alkylengruppe. Besonders bevorzugt steht Y für eine $C_1$- bis $C_4$-Alkylengruppe, insbesondere eine $C_1$-Alkylengruppe, die durch ein oder zwei Carboxyl-, bzw. Carboxylataniongruppen, substituiert sein kann.

X steht bevorzugt für eine Gruppe -(N-$CH_3$)- oder -(N-$CH_2$-$CH_3$)-, besonders bevorzugt für eine -NH-Gruppe. Z steht bevorzugt für ein Wasserstoffatom oder eine Methylgruppe.

Die erfindungsgemäßen Phenone sind nach dem erfindungsgemäßen Verfahren in einfacher Weise durch Umsetzung einer Nitril-funktionalisierten Verbindung der allgemeinen Formel IV

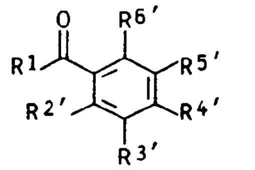

IV

mit einer Verbindung der allgemeinen Formel V

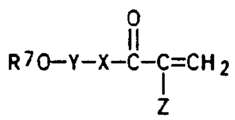

V

erhältlich, wobei $R^1$ die oben genannten Bedeutung hat, $R^7$ für ein Wasserstoffatom oder eine $C_1$-$C_4$-Alkylgruppe steht und $R^{2'}$ bis $R^{6'}$ für ein Wasserstoffatom, eine $C_1$-$C_4$-Alkyl, Alkoxy- oder Thioalkylgruppe stehen, wobei $R^{3'}$, $R^{4'}$ und $R^{5'}$ zusätzlich für eine Hydroxylgruppe stehen können, $R^{2'}$ und $R^{6'}$ zusätzlich gemeinsam mit $R^1$ eine Ethylen- oder Propylenbrücke bilden können und mindestens einer, höchstens jedoch drei der Reste $R^{2'}$ bis $R^{6'}$ für einen Rest -C≡N oder -K-C≡N steht, wobei K die in Anspruch 1 genannte Bedeutung hat und X, Y und Z ebenfalls die in Anspruch 1 genannte Bedeutung haben.

Die Umsetzung verläuft nach folgendem einfachen Reaktionsschema, verdeutlicht beispielsweise für den Fall, daß $R^{4'}$ -K-C≡N oder C≡N ist.

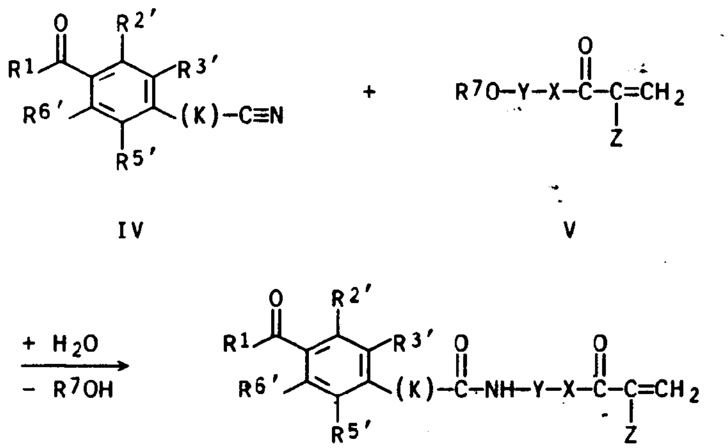


Die Umsetzung wird bevorzugt in einem Gemisch von Schwefelsäure und Essigsäure bei Raumtemperatur durchgeführt. Zur Verhinderung einer vorzeitigen Polymerisation wird gemäß einer bevorzugten Ausführungsform ein Polymerisationsinhibitor, z.B. Nitrobenzol, Chinon, Hydrochinonmonomethylether, 2,6-Di-tert.-butyl-p-kresol, Phenothiazin oder Cu-(II)-Salze, wie $CuSO_4$ oder $CuCl_2$ zugegeben.

Eine Polymerisation kann auch durch die Gegenwart von Luftsauerstoff inhibiert werden.

Die Verbindung V wird bevorzugt im Überschuß z.B. in einem 5 bis 15 %igen, molaren Überschuß, bezogen auf Verbindung IV eingesetzt.

Die Reaktionsdauer beträgt im allgemeinen 1 bis 10 h, insbesondere 2 bis 4 h.

Die Umsetzung verläuft schon bei Raumtemperatur in sehr hohen Ausbeuten, im allgemeinen verläuft sie quantitativ.

Die Aufarbeitung der Reaktionslösung erfolgt vorzugsweise durch Zugabe von Wasser oder Eis zum Reaktionsprodukt. Abfiltrierung des Reaktionsprodukts und Umkristallisation aus polaren protischen Lösungsmitteln, z.B. Ethanol.

Eine Überführung von in der Verbindung enthaltenden Carboxylgruppen in Salze z.B. Kalium- oder Natriumcarboxylate kann z.B durch Zugabe der entsprechenden Alkalimetallhydroxide erfolgen.

Bei den Ausgangsverbindungen handelt es sich um an sich bekannte Verbindungen.

Nitrile der allgemeinen Formel IV lassen sich nach bekannten Verfahren entweder durch nukleophile Substitution von Halogenen entsprechender halogenierter Phenonderivate gegen die Cyanidgruppe oder durch Umsetzen von $\alpha$-Chloracetonitril bzw. von $\gamma$-Chlorbutyronitril mit dem Kaliumsalz des entsprechenden Hydroxybenzophenons gewinnen.

Die Herstellung von Verbindungen der Formel V, z.B. von N-(Hydroxymethyl)methacrylamid, ist bei H. Feuer und U.E. Lynch, J. Am. Chem. Soc. 75, S. 5027 (1953) oder in der US-A-3 064 050 beschrieben. N-(Hydroxymethyl)methacrylamid ist demnach durch Umsetzung von Methacrylamid mit Paraformaldehyd in wasserfreiem Tetrachlorkohlenstoff erhältlich.

N-(Hydroxymethyl)acrylamid ist z.B. durch eine analoge Umsetzung in wasserfreiem Ethylenchlorid erhältlich.

Ungesättigte Carbonamid-N-methylalkylether sind z.B. weiterhin nach E. Müller, K. Dinges und W. Graulich, Makromol. Chem. 57, S. 27 (1962) durch Einwirkung von Alkanolen auf die entsprechende Methylolverbindung zugänglich.

Die erfindungsgemäßen, ungesättigten Phenonderivate weisen in der Regel ein voll befriedigendes Kristallisationsverhalten auf und sind bei Zimmertemperatur üblicherweise fest, so daß sie durch Umkristallisieren leicht in hoher Reinheit erhältich sind. Sie eignen sich insbesondere als copolymerisierbare Monomere zur Herstellung von Polymerisaten, die nach Einwirkung von aktinischer Strahlung eine erhöhte innere Festigkeit aufweisen. Sie können jedoch auch mit sich selbst polymerisiert werden. Bemerkenswerterweise weisen sie insbesondere im kurz- bis längerwelligen UV-Bereich, 250 bis 400 nm, eine erhöhte photochemische Reaktivität auf.

Bei der Verwendung der erfindungsgemäßen Phenone zur Herstellung von Homo- oder Copolymerisaten können die üblichen Verfahren der Vinylpolymerisation benutzt werden. Beispielsweise können solche Polymerisate durch radikalische Polymerisation in Masse, Suspension, Lösung oder Emulsion erhalten werden. Die Polymerisation der ungesättigten Phenone kann aber auch durch ionische Katalystoren oder durch stereospezifische Katalysatoren vom Ziegler-Typ bewirkt werden.

Vorzugsweise erfolgt die Polymerisation radikalisch initiiert. Die Bindung des Phenongerüsts an das Polymerrückgrat der Polymerisate weist eine erhöhte thermische Stabilität auf, wodurch die Polymerisate auch bei erhöhten Temperaturen in befriedigender Weise verarbeitet werden können. Die Phenone sind insbesondere für radikalisch initiierte wäßrige Emulsionspolymerisationsverfahren geeignet.

Besonders geeignete Comonomere zu den erfindungsgemäßen Phenonderivaten sind beispielsweise Styrol, $\alpha$-Methylstyrol, Acrylsäure- und Methacrylsäureester von 1 bis 24 C-Atome enthaltenden Alkanolen wie Methylacrylat, Methylmethacrylat, Ethylacrylat, Ethylmethacrylat, n-Propylacrylat, n-Propylmethacrylat, n-, tert.- und iso-Butylacrylat und -methacrylat, 2-Ethylhexylacrylat und -methacrylat, iso-Amylacrylat, n-Heptylacrylat, iso-Octylacrylat, Isobornylmethacrylat und Isobornylacrylat, des weiteren Vinylester aliphatischer Carbonsäuren, die 2 bis 18 C-Atome enthalten, wie z.B. Vinylacetat oder Vinylpropionat, sowie Acrylamid, Methacrylamid, N-Vinylpyrrolidon, Vinylimidazol, N-Vinylformamid, N-Vinylcaprolactam, Acrylsäure, Methacrylsäure, Acrylnitril, Methacrylnitril, Maleinsäureanhydrid, Itaconsäureanhydrid, Maleinsäure und Fumarsäure, Diester, Diamide und Imide olefinisch ungesättigter Dicarbonsäuren wie z.B. Maleinsäurediimid, Dimethylmaleinat, Dimethylfumarat, Di-n-butylmaleinat oder Di-n-butylfumarat, sowie die Halbester und Halbamide olefinisch ungesättigter Dicarbonsäuren wie Mono-n-butylmaleinat und Mono-n-butylmaleinsäureamid, aber auch Monomere wie Vinylchlorid, Vinylidenchlorid, Vinylfluorid, Ethen, Propen, Butadien, Diallylphthalat und Isopren sowie Gemische der genannten Monomeren. Vorzugsweise werden monoethylenisch ungesättigte Verbindungen als Comonomere eingesetzt.

Die erfindungsgemäßen Phenone sind in den genannten Comonomeren im allgemeinen unter den Copolymerisationsbedingungen ausreichend löslich. Bei Verwendung von Carboxylgruppen oder Carboxylatgruppen enthaltenden Phenonen, empfiehlt sich ein Anlösen entweder in ebenfalls sehr polaren Monomeren wie beispielsweise Methylacrylat oder Acrylsäure oder in geringen Mengen sehr polarer Lösemittel.

Insbesondere eignen sich die Phenone auch zur Herstellung von Propfcopolymerisaten, wobei das Phenon in Gegenwart von zuvor hergestellten Vinylpolymeren wie Polyvinylhalogeniden oder Polyvinylestern oder anderen Polymerisaten wie Polyolefinen polymerisiert wird.

Unabhängig vom Polymerisationsverfahren, nach dem die die Phenone einpolymerisiert enthaltenden Polymerisate hergestellt werden, zeigen diese stets eine Empfindlichkeit gegenüber aktinischer Strahlung, insbesondere im Wellenlängenbereich von 250 bis 400 nm, und weisen nach deren Einwirkung eine erhöhte innere Festigkeit auf. Diese erhöhte innere Festigkeit zeigt sich z.B. durch eine erhöhte Steifigkeit, einen erhöhten Schmelzpunkt sowie eine reduzierte Löslichkeit des Polymerisats in den verschiedensten Lösungsmitteln und einer daraus resultierenden erhöhten Beständigkeit gegenüber Ölen, Fetten, Wasser und dergleichen. Diese Eigenschaften sind vielfach erwünscht, so z.B. bei der Verwendung von Polymerisaten bei Photoreproduktionsverfahren. Aber auch bei der Verwendung solcher Polymerisate zum Beschichten oder Imprägnieren sind diese Eigenschaften von Vorteil. Üblicherweise wird das die Phenone enthaltende Polymerisat erst nach seiner Verformung zu einem Film, einem Überzug oder einem anderen Gegenstand, aktinischer Strahlung ausgesetzt.

Insbesondere eignen sich Polymerisate bzw. Copolymerisate der erfindungsgemäßen Phenone für die Verwendung als Haftklebstoffe, da sie durch Bestrahlung mit energiereicher Strahlung vernetzbar sind und so eine erhöhte innere Festigkeit aufweisen und eine gute Haftung auf Substratoberflächen besitzen. Außerdem zeigen sie nach Bestrahlung eine erhöhte Schälfestigkeit. Der Gewichtsanteil der Phenone in den Copolymerisaten beträgt bei der Verwendung als Haftklebstoffe im allgemeinen 0,01 bis 50 Gew.-%. Bevorzugt beträgt er 0,1 bis 10 Gew.-%, bezogen auf das Copolymerisat. Besonders bevorzugt sind die Copolymerisate für die Verwendung als Haftklebestoffe aufgebaut aus:

a) 0,25 bis 5 Gew.-% wenigstens eines erfindungsgemäßen Phenons und

b) 95 bis 99,75 Gew.-% wenigstens eines copolymerisierbaren monoethylenisch ungesättigten Monomeren.

Von besonderem Interesse für Haftkleber sind Copolymerisate, deren Monomerenzusammensetzung so bemessen ist, daß ein nur aus den Monomeren b) aufgebautes Polymerisat eine Glasübergangstemperatur von -45 bis 0°C, besonders bevorzugt von -30 bis -10°C, aufweisen würde. Nach Fox (T.G.Fox, Bull. Am. Phys. Soc. (Ser. II) 1,123 [1956]) gilt für die Glasübergangstemperatur von Mischpolymerisaten in guter Näherung:

$$\frac{1}{T_g} = \frac{X^1}{T_g^1} + \frac{X^2}{T_g^2} + \ldots\ldots\ldots \frac{X^s}{T_g}s$$

wobei $X^1$, $X^2$, ....., $X^s$ die Massenbrüche der Monomeren, 1, 2, ....., s und $T_g^1$, $T_g^2$, .... $T_g^s$ die Glasübergangstemperaturen der jeweils nur aus einem der Monomeren 1, 2, .... oder s aufgebauten Polymeren in Grad Kelvin bedeuten. Die Glasübergangstemperaturen von obengenannten Monomeren b) sind im wesentlichen bekannt und z.B. in J. Brandrup, E. H. Immergut, Polymer Handbook 1St Ed. J. Wiley, New York 1966 und 2nd Ed. J. Wiley, New York 1975 aufgeführt.

Die als Haftklebstoff geeigneten Polymerisate und Copolymerisate weisen vorzugsweise eine Glasübergangstemperatur von -45 bis 0°C, insbesondere von -30 bis -10°C auf.

Darüber hinaus weisen solche als Haftklebstoffe geeignete Polymerisate vor ihrer Bestrahlung in Tetrahydrofuran (THF) bei 25°C vorzugsweise einen K-Wert von 20 bis 70, besonders bevorzugt von 30 bis 55 auf (1 gew.-%ige Lösung in THF).

Der K-Wert ist eine relative Viskositätszahl, die nach DIN 53 726 bestimmt wird. Er charakterisiert das mittlere Molekulargewicht des Polymerisats. Die Anfangsoberflächenklebrigkeit der beschriebenen Haftklebstoffe kann man modifizieren, indem man den erfindungsgemäßen Polymerisaten in Mengen von bis zu 50 Gew.-% klebrigmachende Harze (Tackifier) wie Cumaron-Inden-, Alkylphenol-Formaldehyd- oder Alkydharze zugibt. In untergeordneten Mengen, vorzugsweise nicht mehr als 30, insbesondere nicht mehr als 15 Gew.-%, können den als Haftklebstoffen geeigneten erfindungsgemäßen Polymerisaten vor ihrer Anwendung auch mineralische Füllstoffe, Weichmacher, polychlorierte Kohlenwasserstoffe oder Paraffinöle zugesetzt werden.

Anwendungstechnisch bevorzugt ist die Verwendung der erfindungsgemäßen Haftklebstoffe zur Herstellung selbstklebender Artikel, insbesondere zur Herstellung von Klebebändern und -folien, die ganz allgemein aus einem Trägermaterial und einem Haftklebstoff bestehen. Je nach Anwendungsfall werden die Trägermaterialien aus unterschiedlichen Substraten ausgewählt. Geeignet sind u.a. textile Gewebe, Papiere, Kunststoff-Folien aus Polyvinylchlorid, Polyester wie Polyethylenglycolterephthalat, Celluloseacetat, Polypropylen, Metallfolien aus Aluminium, Kupfer oder Blei, aber auch Schaumstoffe aus Polyurethan, Polyvinylchlorid, Polyethylen sowie Polychloropren. Die Applikation der erfindungsgemäßen Haftklebstoffe erfolgt bevorzugt vor der Bestrahlung mit energiereichem Licht. Sie kann aus organischer Lösung, bevorzugt einen Feststoffgehalt von 50 bis 80 Gew.-% aufweisend, oder aus der Schmelze erfolgen, wobei im Falle der Verwendung organischer Lösungen der erfindungsgemäßen Polymerisate das Lösungsmittel nach dem Beschichten der Trägermaterialoberfläche im allgemeinen mittels Wärme ausgetrieben wird. Vorzugsweise wird aus der Schmelze, bei Temperaturen von 80 bis 140°C, appliziert. Die Applikation kann z.B. durch Streichen, Verdüsen, Walzen, Rakeln oder Gießen erfolgen. Aufgrund der polaren Spacerkette, die das Phenongerüst mit dem Polymerrückgrat verbindet, zeigen die oben beschriebenen Haftklebstoffe eine besondere Adhäsion auf polaren Trägermaterialien.

Die Bestrahlung mit energiereichem, bevorzugt ultraviolettem Licht kann unmittelbar nach dem Auftragen, nach der Entfernung des Lösungsmittels (Applikation aus der Lösung) oder nach Durchlaufen einer Heiz-, Temper- und/oder Kühlstrecke (insbesondere bei Applikation aus der Schmelze) erfolgen. Zum Bestrahlen können handelsübliche UV-Strahler, die vorzugsweise in einem Wellenlängenbereich von 250 bis 400 nm Strahlung emittieren, eingesetzt werden. Geeignet sind beispielsweise Quecksilbermitteldruckstrahler mit einer Strahlungsleistung von 80 bis 120 W/cm, wie sie z.B. in "Sources and Applications of Ultraviolett Radiation", R. Philips, Academic Press, London 1983, beschrieben werden. Die Bestrahlungsdauer richtet sich nach der Dicke der Beschichtung, dem UV-Emissionsspektrum und der Strahlungsleistung der verwendeten Strahlungsquelle sowie den jeweils einpolymerisierten Phenonen I. Sie kann jedoch in Vorversuchen leicht ermittelt werden. Die Bestrahlung erfordert keine Gegenwart von Schutzgas.

Beispiele

Beispiel 1

Herstellung erfindungsgemäßer Phenone

Bei den hergestellten Phenone wurde die Struktur und Aufbau durch $^{1}H$-NMR-, $^{13}C$-NMR-, IR- und Massenspektroskopie sowie durch Elementaranalyse bestimmt.

a) N-(Acrylamidomethyl)-benzophenon-4-carbonsäureamid

Eine Mischung aus 132 g Schwefelsäure, 68 g Essigsäure, 0,10 Mol 4-Cyanobenzophenon, 0,11 Mol N-Methylol-acryl-amid (Amol) und 100 ppm 2,6-Di-tert.-butyl-p-kresol wurde 4 h bei Raumtemperatur gerührt. Der Reaktionsansatz wurde durch Zugabe von gemahlenen Eis hydrolysiert und der entstehende kristalline Niederschlag nach ca. 20 h abgesaugt. Das Produkt wurde mit Wasser gewaschen und an der Luft getrocknet. Die Umkristallisation erfolgte aus Ethanol.

Die Herstellung der Verbindungen b) bis g) erfolgte in entsprechender Weise. In Tabelle 1 sind die Strukturformeln und in Tabelle 2 Analysedaten angegeben.

Tabelle 1

Säurekatalysierte Anlagerung von N-Methylol-acrylamid (= Amol) bzw. α-Acrylamido-glykolsäure (= AGS) an Nitrile

| Verbindung | Strukturformel | Einsatzstoffe |
|---|---|---|
| (a) | 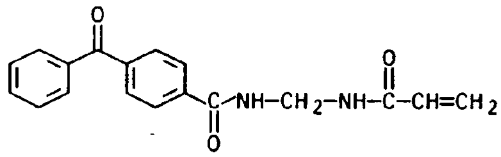 | 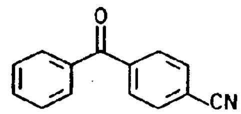 und Amol |
| (b) | 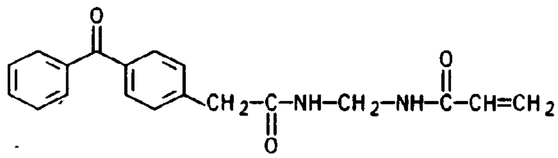 | 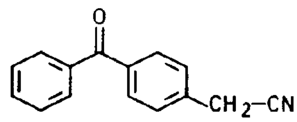 und Amol |
| (c) | 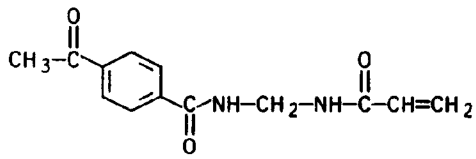 | 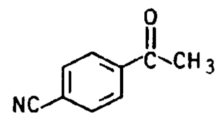 und Amol |
| (d) | 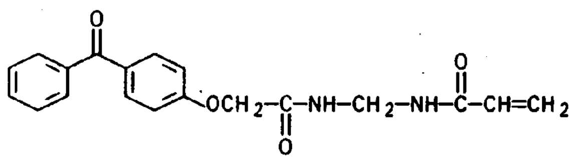 | 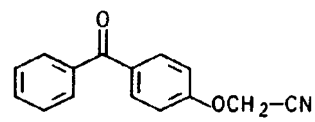 und Amol |
| (e) | 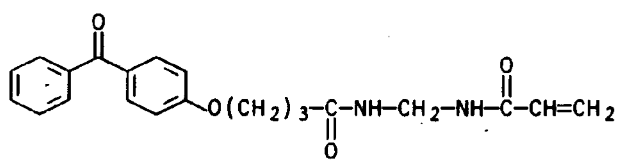 | 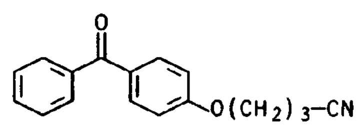 und Amol |
| (f) | 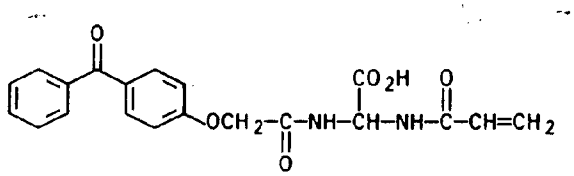 | 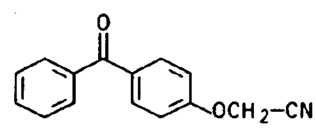 und AGS |
| (g) | 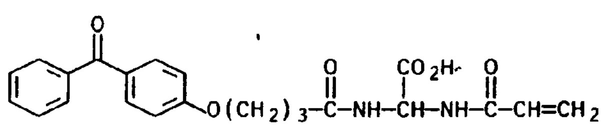 | 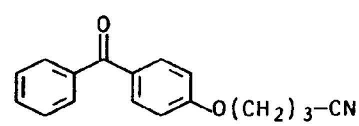 und AGS |

Tabelle 2

Angaben über Herstellung und analytische Daten

| Verbindung | Ausbeute | Umkristallisation aus | Schmelzpunkt | Elementaranalyse | C | H | O | N |
|---|---|---|---|---|---|---|---|---|
| (a) | 85 % | Ethanol | 183-186°C | Ber. | 70,12 | 5,23 | 15,57 | 9,09 |
| | | | | Gef. | 70,1 | 5,2 | 15,9 | 8,8 |
| (b) | 77,5 % | Ethanol | 193-196°C | Ber. | 70,79 | 5,63 | - | 8,69 |
| | | | | Gef. | 70,5 | 5,8 | - | 8,3 |
| (c) | 86 % | Dimethylformamid/ Wasser (1:2) | 197-201°C | Ber. | 63,40 | 5,73 | 19,49 | 11,38 |
| | | | | Gef. | 62,9 | 5,9 | 19,7 | 11,1 |
| (d) | 99 % | Ethanol | 176-178°C | Ber. | 67,45 | 5,36 | 18,91 | 8,28 |
| | | | | Gef. | 67,0 | 5,5 | 19,3 | 8,1 |
| (e) | 93 % | Ethanol | 140°C (Polymerisation) | Ber. | 68,84 | 6,05 | - | 7,65 |
| | | | | Gef. | 68,4 | 6,2 | - | 7,5 |
| (f) | 88 % | Acetonitril | 192-194°C | Ber. | 62,82 | 4,75 | 25,10 | 7,33 |
| | | | | Gef. | 62,6 | 4,9 | 25,3 | 7,2 |
| (g) | 91 % | Reinigung durch Fällung aus der wäßrigen Na-Salz-Lösung mit HCl | 187-191°C | Ber. | 64,38 | 5,40 | - | 6,83 |
| | | | | Gef. | 64,2 | 5,6 | - | 6,9 |

Beispiel 2

Herstellung eines Copolymerisats

50 g einer Monomerenmischung aus

500 g n-Butylacrylat
290 g 2-Ethylhexylacrylat
185 g Methylacrylat
25 g Acrylsäure
6,5 g N-(Acrylamidomethyl)-benzophenon-4-carbonsäureamid

wurden in 150 g Toluol in Gegenwart von 1 g tert.-Butylperoktoat (Polymerisationsinitiator) auf eine Reaktionstemperatur von 80°C erwärmt. Anschließend wurde im Verlauf von 5 h bei dieser Temperatur der Rest der Monomermischung sowie parallel dazu über 3 h eine Lösung von 19 g tert.-Butylperoktoat in 100 g Toluol zugesetzt. Danach wurde bei 120°C nachpolymerisiert und das Lösemittel schließlich destillativ abgetrennt.

Es wurde ein bei Raumtemperatur fließfähiges Polymerisat erhalten, das in THF (25°C) einen K-Wert von 44 hatte.

Beispiel 3

Prüfung der Haftklebeeigenschaften des Polymerisats aus Beispiel 2.

a) Herstellung der Prüfstreifen

Zur Herstellung der Prüfstreifen wurde das Polymerisat aus der Schmelze in einer Schichtdicke von 25 g/m$^2$ auf eine Polyesterfolie als Träger aufgetragen.

Anschließend wurde die Polyesterfolie mit einer Geschwindigkeit von 20 m/min im Abstand von 10 cm unter zwei hintereinander (im Abstand von 11 cm) angeordneten Quecksilbermitteldruckstrahlern (80 W/cm) durchgeführt. Aus der so erhaltenen selbstklebenden Folie wurden Streifen von 2 cm Breite und 5 cm Länge ausgeschnitten.

b) Prüfung der Scherstandfestigkeit

Die Prüfstreifen wurden auf einer Länge von 2,5 cm, unter Anwendung eines Gewichtes der Masse 2,5 kg, auf ein chromiertes Stahlblech (V2A) in der Weise aufgebracht, daß sie zu einer Seite überstanden und die gegenüberliegende Seite nicht beschichtet war. Das Stahlblech wurde dann 24 h bei 20°C und Normaldruck gelagert. Anschließend wurde das nicht behaftete Ende des Stahlblechs zwischen 2 Klemmbacken befestigt und das gegenüberliegende überstehende Klebeband frei hängend bei Temperaturen von 25° mit einem Gewicht der Masse 2 kg und bei 50°C mit einem Gewicht der Masse 1 kg belastet. Maßstab für die Scherstandfestigkeit ist die Zeitdauer bis zum Aufbrechen des Klebstoffilms. Zum Vergleich wurde die Prüfung mit nicht bestrahlten Polyesterfolien wiederholt. Die Ergebnisse zeigt Tabelle 3.

c) Prüfung der Schälfestigkeit

Zur Bestimmung der Schälfestigkeit der Prüfstreifen auf der Oberfläche eines Substrates, wurden diese auf einer Länge von 2,5 cm, unter Anwendung eines Gewichtes der Masse 2,5 kg, auf ein chromiertes Stahlblech (V2A) aufgerollt. 24 h danach (Lagerung bei 20°C und Normaldruck) wurde die Kraft bestimmt, um die Prüfstreifen in einer Zugdehnungsprüfapparatur bei einem Abschälwinkel von 180°C mit einer Geschwindigkeit von 300 mm/min rückwärts abzuziehen. Die Ergebnisse enthält ebenfalls Tabelle 3.

Tabelle 3

| | Scherstandfestigkeit [h] | | Schälfestigkeit [N/cm] |
|---|---|---|---|
| | 25°C | 50°C | |
| mit Bestrahlung | >24 | >24 | 4,3 |
| ohne Bestrahlung | < 1 | < 1 | - |

**Patentansprüche**

1. Ungesättigte Phenonderivate der allgemeinen Formel I

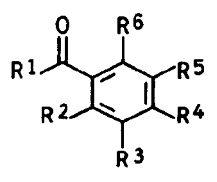

I

in der die Variablen folgende Bedeutung haben:

| | |
|---|---|
| $R^1$ | eine $C_1$- bis $C_4$-Alkylgruppe, eine Cyclopropyl-, -pentyl- oder -hexylgruppe, eine Indanonyl- oder Tetralonylgruppe, die Phenylgruppe, eine Phenylgruppe, deren Wasserstoffatome teilweise oder vollständig durch eine $C_1$- bis $C_4$-Alkyl-, -Alkoxy- oder -Thioalkylgruppe ersetzt sind, oder gemeinsam mit $R^2$ oder $R^6$ eine Ethylen- oder Propylenbrücke; |
| $R^2$ bis $R^6$ | ein Wasserstoffatom, eine $C_1$- bis $C_4$-Alkyl, -Alkoxy- oder -Thioalkylgruppe, wobei $R^3$, $R^4$ und $R^5$ zusätzlich für eine Hydroxylgruppe stehen können, $R^2$ oder $R^6$ zusätzlich gemeinsam mit $R^1$ eine Ethylen- oder Propylenbrücke bilden können und mindestens einer, höchstens jedoch drei der Reste $R^2$ bis $R^6$ für eine Gruppe der allgemeinen Formel II |

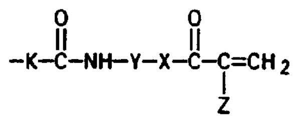

II

oder III

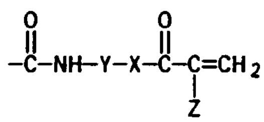

III

stehen, in denen die Variablen folgende Bedeutung haben:

| | |
|---|---|
| K | eine $C_1$- bis $C_{10}$-Alkylen-, eine 1 bis 2 Sauerstoff- oder Schwefelatome enthaltende $C_1$- bis $C_{10}$-Alkylengruppe |
| Y | eine geradlinige oder verzweigte $C_1$- bis $C_{10}$-Alkylengruppe oder eine durch Carboxyl-, Carboxylatanion-, $C_1$- bis $C_4$-Carbonsäurealkylester- oder Hydroxygruppen substituierte $C_1$- bis $C_{10}$-Alkylengruppe |
| X | -NH- oder -(N-Alkyl)- mit 1 bis 4 C-Atomen |
| Z | Wasserstoff oder eine $C_1$- bis $C_4$-Alkylgruppe |

**2.** Phenonderivate nach Anspruch 1, die als $R^1$ die Phenyl- oder eine $C_1$-bis $C_4$-Alkylgruppe, als Reste $R^2$, $R^3$, $R^5$ und $R^6$ ein Wasserstoffatom und als $R^4$ eine Gruppe der allgemeinen Formel II oder III, in der K für eine $C_1$- bis $C_4$-Alkylen- oder eine $C_1$- bis $C_4$-Oxyalkylengruppe, bei der das Sauerstoffatom direkt an den Phenylring gebunden ist, Y für eine $C_1$- bis $C_4$-Alkylen- oder eine durch ein oder zwei Carboxyl-, Carboxylatanion-, $C_1$- bis $C_4$-Carbonsäurealkylester- oder Hydroxylgruppen substituierte $C_1$- bis $C_4$-Alkylengruppe, X für eine -NH-, $-(N-CH_3)$-oder $-(N-CH_2-CH_3)$- Gruppe und Z für ein Wasserstoffatom oder eine Methylgruppe steht, enthalten.

**3.** Homo- oder Copolymerisate, die Phenonderivate gemäß einem der Ansprüche 1 oder 2 einpolymerisiert enthalten.

**4.** Copolymerisate, die 0,1 bis 10 Gew.-% wenigstens eines Phenonderivats gemäß den Ansprüchen 1 oder 2 einpolymerisiert enthalten.

**5.** Copolymerisate, die aus

a) 0,25 bis 5 Gew.-% wenigstens eines Phenonderivate gemäß den Ansprüchen 1 oder 2 und

b) 95 bis 99,75 Gew.-% wenigstens eines copolymerisierbaren Monomeren

aufgebaut sind, eine Glasübergangstemperatur von -45 bis 0°C und in Tetrahydrofuran bei 25°C einen K-Wert von 20 bis 70 aufweisen.

**6.** Verfahren zur Herstellung von ungesättigten Phenonderivaten gemäß den Ansprüchen 1 oder 2, dadurch gekennzeichnet, daß man eine Verbindung der allgemeinen Formel IV

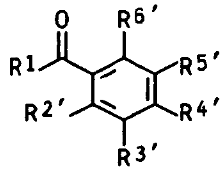

IV

mit einer Verbindung der allgemeinen Formel V

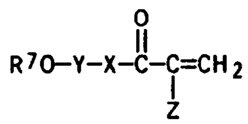

V

umsetzt, wobei $R^1$ die oben genannten Bedeutung hat, $R^7$ für ein asserstoffatom oder eine $C_1$-$C_4$-Alkylgruppe steht und $R^{2'}$ bis $R^{6'}$ ein Wasserstoffatom, eine $C_1$-$C_4$-Alkyl, -Alkoxy-, oder -Thioalkylgruppe, wobei $R^{3'}$, $R^{4'}$ und $R^{5'}$ zusätzlich für eine Hydroxylgruppe stehen können, $R^{2'}$ und $R^{6'}$ zusätzlich gemeinsam mit $R^1$ eine Ethylen- oder Propylenbrücke bilden können und mindestens einer, höchstens jedoch drei der Reste $R^{2'}$ bis $R^{6'}$ für einen Rest -C≡N oder -K-C≡N steht, wobei K die in Anspruch 1 genannte Bedeutung hat und X, Y und Z ebenfalls die in Anspruch 1 genannte Bedeutung haben.

7. Verwendung von Copolymerisaten gemäß den Ansprüchen 3 bis 5 als Haftklebstoffe.

8. Verfahren zur Herstellung selbstklebender Artikel dadurch gekennzeichnet, daß ein Trägermaterial mit einer organischen Lösung oder der Schmelze eines Copolymerisats gemäß den Ansprüchen 3 bis 5 beschichtet und danach mit energiereicher Strahlung bestrahlt wird.

9. Selbstklebende Artikel erhältlich unter Verwendung von Copolymerisaten gemäß Ansprüchen 3 bis 5.

## Claims

1. An unsaturated phenone derivative of the formula I

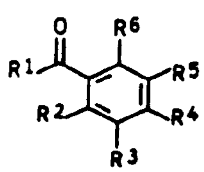

I

where
$R^1$ is $C_1$-$C_4$-alkyl, cyclopropyl, cyclopentyl, cyclohexyl, indanonyl, tetralonyl, phenyl or phenyl in which some or all of the hydrogen atoms have been replaced by a $C_1$-$C_4$-alkyl, $C_1$-$C_4$-alkoxy or $C_1$-$C_4$-thioalkyl group, or together with $R^2$ or $R^6$ forms an ethylene or propylene bridge,
$R^2$ to $R^6$ are each hydrogen, $C_1$-$C_4$-alkyl, $C_1$-$C_4$-alkoxy or $C_1$-$C_4$-thioalkyl, and $R^3$, $R^4$ and $R^5$ may each additionally be hydroxyl, $R^2$ or $R^6$ may additionally together with $R^1$ form an ethylene or propylene bridge and one or more, but not more than three, of the radicals $R^2$ to $R^6$ are a group of the formula II

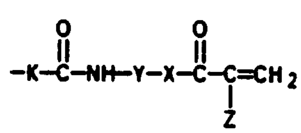

II

or III

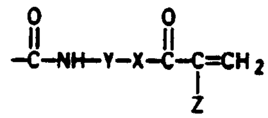

III

where
K is $C_1$-$C_{10}$-alkylene which may contain 1 or 2 oxygen or sulfur atoms,
Y is straight-chain or branched $C_1$-$C_{10}$-alkylene or is $C_1$-$C_{10}$-alkylene which is substituted by carboxyl, a carboxylate anion, an alkyl $C_1$-$C_4$-carboxylate group or hydroxyl, X is -NH- or -(N-alkyl)- of 1 to 4 carbon atoms and
Z is hydrogen or $C_1$-$C_4$-alkyl.

2. A phenone derivative as claimed in claim 1, wherein $R^1$ is phenyl or $C_1$-$C_4$-alkyl, $R^2$, $R^3$, $R^5$ and $R^6$ are each hydrogen and $R^4$ is a group of the formula II or III, where K is $C_1$-$C_4$-alkylene or $C_1$-$C_4$-oxyalkylene in which the oxygen atom is bonded directly to the phenyl ring, Y is $C_1$-$C_4$-alkylene which may be substituted by one or two carboxyl groups, carboxylate anions, alkyl $C_1$-$C_4$-carboxylate groups or hydroxyl groups, X is -NH-, -(N-$CH_3$)- or -(N-$CH_2$-$CH_3$)- and Z is hydrogen or methyl.

3. A homo- or copolymer which contains, as polymerized units, a phenone derivative as claimed in claim 1 or 2.

4. A copolymer which contains, as polymerized units, from 0.1 to 10% by weight of one or more phenone derivatives as claimed in claim 1 or 2.

5. A copolymer which is composed of
a) from 0.25 to 5% by weight of one or more phenone derivatives as claimed in claim 1 or 2 and
b) from 95 to 99.75% by weight of one or more copolymerizable monomers,
has a glass transition temperature of from -45 to 0°C and possesses a K value of from 20 to 70 in tetrahydrofuran at 25°C.

6. A process for the preparation of an unsaturated phenone derivative as claimed in claim 1 or 2, wherein a compound of the formula IV

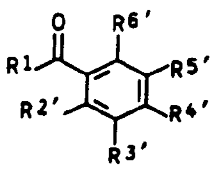

IV

is reacted with a compound of the formula V

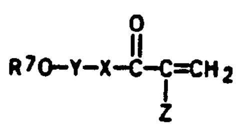

V

where $R^1$ has the abovementioned meanings, $R^7$ is hydrogen or $C_1$-$C_4$-alkyl and $R^{2'}$ to $R^{6'}$ are each hydrogen, $C_1$-$C_4$-alkyl, $C_1$-$C_4$-alkoxy or $C_1$-$C_4$-thioalkyl, and $R^{3'}$, $R^{4'}$ and $R^{5'}$ may additionally be hydroxyl, $R^{2'}$ and $R^{6'}$ together with $R^1$ may additionally form an ethylene or propylene bridge and one or more, but not more than three, of the radicals $R^{2'}$ to $R^{6'}$ are a radical -C≡N or -K-C≡N, where K, X, Y and Z have the meanings stated in claim 1.

7. Use of a copolymer as claimed in claim 3 or 4 or 5 as a contact adhesive.

8. Process for the production of a self-adhesive article, wherein a substrate is coated with an organic solution or the melt of a copolymer as claimed in claim 3 or 4 or 5 and is then exposed to high energy radiation.

9. A self-adhesive article obtainable using a copolymer as claimed in claim 3 or 4 or 5.

## Revendications

1. Dérivés de phénone insaturés de la formule générale I

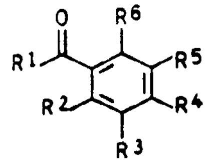

I

dans laquelle les diverses variables ont les significations suivantes :

$R^1$ représente un radical alkyle en $C_1$-$C_4$, un radical cyclopropyle, cyclopentyle ou cyclohexyle, un radical indanonyle ou tétralonyle, le radical phényle, un radical phényle dont les atomes d'hydrogène sont totalement ou partiellement remplacés par des radicaux alkyle en $C_1$-$C_4$, alcoxy en $C_1$-$C_4$ ou thioalkyle $C_1$-$C_4$, ou forme un pont éthylène ou propylène en commun avec $R^2$ ou $R^6$,

$R^2$ à $R^6$ représentent chacun un atome d'hydrogène, un radical alkyle en $C_1$ à $C_4$, alcoxy en $C_1$ à $C_4$ ou thioalkyle en $C_1$ à $C_4$, où $R^3$, $R^4$ et $R^5$ peuvent représenter chacun complémentairement un groupe hydroxyle, $R^2$ ou $R^6$ peuvent complémentairement représenter en commun avec $R^1$ un pont éthylène ou propylène et au moins un, mais au maximum cependant trois, des symboles $R^2$ à $R^6$ représentent chacun un groupe de la formule générale II

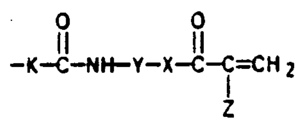

II

ou III

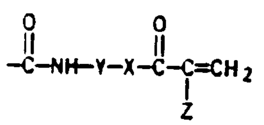

III

où les diverses variables ont les significations suivantes

K représente un radical alkylène en $C_1$ à $C_{10}$, un radical alkylène en $C_1$ à $C_{10}$ contenant un ou deux atomes d'oxygène ou de soufre,

Y représente un radical alkylène en $C_1$ à $C_{10}$, linéaire ou ramifié, ou un radical alkylène en $C_1$ à $C_{10}$ substitué à des radicaux carboxyle, anion carboxylate, ester alkylique d'acide carboxylique en $C_1$ à $C_{10}$, ou hydroxyle,

X représente un radical -NH- ou -(N-alkyle)- comportant de 1 à 4 atomes de carbone,

Z représente un atome d'hydrogène ou un radical alkyle en $C_1$ à $C_4$.

2. Dérivés de phénone suivant la revendication 1, qui contiennent comme $R^1$, le radical phényle ou un radical alkyle en $C_1$ à $C_4$, comme restes $R^2$, $R^3$, $R^5$ et $R^6$, des atomes d'hydrogène et, comme $R^4$, un groupe de la formule générale II ou III dans laquelle K représente un radical alkylène en $C_1$ à $C_4$ ou un radical oxyalkylène en $C_1$ à $C_4$, où l'atome d'oxygène est directement lié au noyau phényle, Y représente un radical alkylène en $C_1$ à $C_4$ ou un radical alkylène en $C_1$ à $C_4$ substitué par un ou deux radicaux carboxyle, anion carboxylate, ester alkylique d'acide carboxylique en $C_1$ à $C_4$, ou hydroxyle, X représente un groupe -NH-, -($N-CH_3$)- ou -($N-CH_2-CH_3$)- et Z représente un atome d'hydrogène ou le radical méthyle.

3. Homopolymères ou copolymères qui contiennent des dérivés de phénone suivant l'une quelconque des

revendications 1 et 2, incorporés par polymérisation.

4. Copolymères qui contiennent 0,1 à 10% en poids d'au moins un dérivé de phénone suivant l'une quelconque des revendications 1 et 2, incorporés par polymérisation.

5. Copolymères, qui sont constitués de
   a) 0,25 à 5% en poids d'au moins un dérivé de phénone suivant l'une quelconque des revendications 1 et 2 et
   b) 95 à 99,75% en poids d'au moins un monomère copolymérisable,

   qui présentent une température de transition vitreuse de -45 à 0°C et qui manifestent une valeur K de 20 à 70 dans le tétrahydrofuranne et à 25°C.

6. Procédé de préparation de dérivés de phénone insaturés suivant l'une quelconque des revendications 1 et 2, caractérisé en ce que l'on fait réagir un composé de la formule générale IV

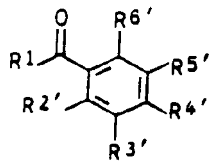

IV

avec un composé de la formule générale V

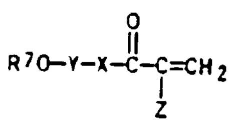

V

où $R^1$ possède les significations qui lui ont été précédemment attribuées, $R^7$ représente un atome d'hydrogène ou un radical alkyle en $C_1$-$C_4$ et $R^{2'}$ à $R^{6'}$ représentent chacun un atome d'hydrogène, un radical alkyle en $C_1$ à $C_4$, alcoxy en $C_1$ à $C_4$, ou thioalkyle en $C_1$ à $C_4$, où $R^{3'}$, $R^{4'}$ et $R^{5'}$ peuvent complémentairement chacun représenter un radical hydroxyle, $R^{2'}$ et $R^{6'}$ peuvent complémentairement former en commun avec $R^1$ un pont éthylène ou propylène et au moins l'un , mais au maximum cependant trois, des restes $R^{2'}$ à $R^{6'}$ représentent des radicaux -C≡-N ou -K-C≡N où K possède les significations qui lui ont été attribuées dans la revendication 1 et X, Y et Z possèdent également les significations qui leur ont été attribuées dans la revendication 1.

7. Utilisation de copolymères suivant l'une quelconque des revendications 3 à 5 à titre de colles de contact.

8. Procédé de fabrication d'articles autocollants, caractérisés en ce que l'on revêt un matériau de support d'une solution organique ou d'une masse fondue d'un copolymère suivant l'une quelconque des revendications 3 à 5 et en ce qu'on l'irradie ensuite à l'aide d'un rayonnement riche en énergie.

9. Articles autocollants obtenus en recourant à 1' emploi de copolymères suivant l'une quelconque des revendications 3 à 5.